# FASCICULE DE BREVET EUROPEEN

(11) **EP 1 642 897 B1**
(45) Date de publication et mention de la délivrance du brevet: **15.10.2008**
(21) Numéro de dépôt: 05291297.9
(22) Date de dépôt: 17.06.2005
(51) Int. Cl.: C07D 333/38, A61K 31/381

(54) **Forme cristalline alpha du ranelate de strontium, son procede de preparation, et les compositions pharmaceutiques qui la contiennent**
Alpha-Kristallform von Strontiumranelat, Verfahren zu ihrer Herstellung und dieser enthaltende pharmazeutische Zusammensetzungen
Alpha-crystalline form of Strontium Ranelate, process of preparation therof and pharmaceutical compositions containing it

(30) Priorité: 30.09.2004 FR 0410335
(43) Date de publication de la demande: 05.04.2006
(60) Demande divisionnaire: 08007270.5
(73) Titulaire: Les Laboratoires Servier, 92415 Courbevoie Cedex (FR)
(72) Inventeur: Horvath, Stéphane, 45380 La Chapelle-Saint-Mesmin (FR); Demuynck, Isabelle, 45000 Orleans (FR); Damien, Gérard, 45130 Meung-sur-Loire (FR)
(74) Mandataire: Giudicelli, Cathy

(56) Documents cités:
- EP-A- 0 415 850
- EP-A- 0 813 869
- WO-A-20/04029036
- SORBERA L A ET AL: "STRONTIUM RANELATE TREATMENT AND PREVENTION OF OSTEOPOROSIS BONE RESORPTION INHIBITOR BONE FORMATION STIMULANT" DRUGS OF THE FUTURE, BARCELONA, ES, vol. 28, no. 4, 1 avril 2003 (2003-04-01), pages 328-335, XP008036784 ISSN: 0377-8282
- REGINSTER J-Y ET AL: "STRONTIUM RANELATE: A NEW PARADIGM IN THE TREATMENT OF OSTEOPOROSIS" DRUGS OF TODAY / MEDICAMENTOS DE ACTUALIDAD, J.R. PROUS SS.A. INTERNATIONAL PUBLISHERS, ES, vol. 39, no. 2, 1 février 2003 (2003-02-01), pages 89-101, XP008036782 ISSN: 0025-7656

## Description

La présente invention concerne la forme cristalline alpha du ranélate de strontium, son procédé de préparation ainsi que les compositions pharmaceutiques qui la contiennent.

Le ranélate de strontium, représenté par la formule (I) : 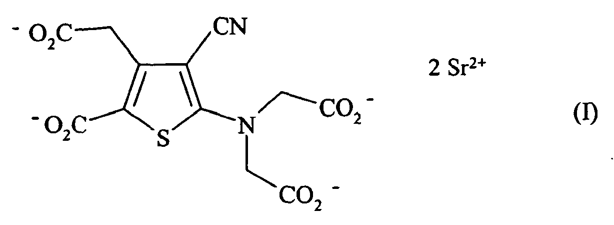 ou sel distrontique de l'acide 5-[bis(carboxyméthyl)amino]-3-carboxyméthyl-4-cyano-2-thiophènecarboxylique, ainsi que ses hydrates, possèdent des propriétés pharmacologiques et thérapeutiques très intéressantes, notamment des propriétés anti-ostéoporotiques remarquables, qui rendent ces composés utiles dans le traitement et la prévention des maladies osseuses.

Le ranélate de strontium, ainsi que ses hydrates, ont également des propriétés qui les rendent utiles dans le traitement et la prévention de l'arthrose.

La préparation et l'utilisation en thérapeutique du ranélate de strontium et de ses tétrahydrate, heptahydrate et octahydrate ont été décrits dans le brevet européen EP 0415 850.

L'utilisation du ranélate de stronium dans la prévention et le traitement de l'arthrose a été décrite dans le brevet européen EP 0 813 869.

La demanderesse a présentement trouvé que le ranélate de strontium pouvait être obtenu sous une forme cristalline bien définie, parfaitement reproductible et présentant de ce fait des caractéristiques intéressantes de filtration et de facilité de formulation.

Plus spécifiquement, la présente invention concerne la forme cristalline alpha du ranélate de strontium, caractérisée par une teneur en eau de 24% et par le diagramme de diffraction X sur poudre suivant, mesuré sur un diffractomètre PANalytical X'Pert Pro avec un détecteur X'Celerator, et exprimé en termes de position de raie (angle de Bragg 2 thêta, exprimé en degrés), de hauteur de raie (exprimée en coups), de surface de raie (exprimée en coups x degrés), de largeur des raies à mi-hauteur ("FWHM", exprimée en degrés) et de distance inter-réticulaire d (exprimée en Å) :

| **Raie n°** | **Angle 2 thêta (degrés)** | **Hauteur (coups)** | **Surface (coups x degrés)** | **FWHM (degrés)** | **Distance inter-réticulaire (Å)** |
|---|---|---|---|---|---|
| 1 | 7,6 | 4527 | 448 | 0,1004 | 11,649 |
| 2 | 8,0 | 1438 | 142 | 0,1004 | 11,069 |
| 3 | 8,3 | 3522 | 349 | 0,1004 | 10,642 |
| 4 | 8,6 | 11347 | 1123 | 0,1004 | 10,272 |
| 5 | 8,9 | 7332 | 726 | 0,1004 | 9,889 |
| 6 | 11,0 | 1047 | 104 | 0,1004 | 8,072 |
| 7 | 11,3 | 1655 | 164 | 0,1004 | 7,840 |
| 8 | 12,0 | 2186 | 216 | 0,1004 | 7,355 |
| 9 | 13,2 | 2887 | 381 | 0,1338 | 6,703 |
| 10 | 13,5 | 1705 | 169 | 0,1004 | 6,557 |
| 11 | 14,1 | 154 | 30 | 0,2007 | 6,275 |
| 12 | 14,7 | 803 | 79 | 0,1004 | 6,035 |
| 13 | 14,9 | 1346 | 178 | 0,1338 | 5,942 |
| 14 | 15,8 | 1556 | 154 | 0,1004 | 5,613 |
| 15 | 16,0 | 3339 | 441 | 0,1338 | 5,527 |
| 16 | 16,7 | 1845 | 183 | 0,1004 | 5,308 |
| 17 | 17,3 | 2835 | 281 | 0,1004 | 5,127 |
| 18 | 17,6 | 1252 | 124 | 0,1004 | 5,049 |
| 19 | 18,0 | 2183 | 216 | 0,1004 | 4,939 |
| 20 | 19,2 | 2303 | 228 | 0,1004 | 4,622 |
| 21 | 19,8 | 1298 | 128 | 0,1004 | 4,475 |
| 22 | 20,3 | 788 | 78 | 0,1004 | 4,373 |
| 23 | 20,6 | 1039 | 103 | 0,1004 | 4,317 |
| 24 | 21,1 | 882 | 116 | 0,1338 | 4,211 |
| 25 | 21,7 | 390 | 38 | 0,1004 | 4,103 |
| 26 | 22,3 | 1919 | 253 | 0,1338 | 3,990 |
| 27 | 22,7 | 1805 | 179 | 0,1004 | 3,923 |
| 28 | 23,0 | 4043 | 467 | 0,1171 | 3,861 |
| 29 | 23,5 | 650 | 86 | 0,1338 | 3,792 |
| 30 | 24,0 | 8677 | 1002 | 0,1171 | 3,711 |
| 31 | 24,7 | 229 | 30 | 0,1338 | 3,600 |
| 32 | 25,1 | 1246 | 164 | 0,1338 | 3,543 |
| 33 | 25,6 | 1659 | 219 | 0,1338 | 3,473 |
| 34 | 25,9 | 1773 | 175 | 0,1004 | 3,442 |
| 35 | 26,3 | 695 | 69 | 0,1004 | 3,385 |
| 36 | 26,6 | 401 | 46 | 0,1171 | 3,355 |
| 37 | 27,0 | 2800 | 370 | 0,1338 | 3,300 |
| 38 | 27,6 | 1415 | 140 | 0,1004 | 3,230 |
| 39 | 28,0 | 3250 | 429 | 0,1338 | 3,186 |
| 40 | 28,4 | 1513 | 250 | 0,1673 | 3,144 |
| 41 | 29,1 | 1456 | 144 | 0,1004 | 3,068 |
| 42 | 29,6 | 1943 | 192 | 0,1004 | 3,022 |
| 43 | 30,1 | 3637 | 540 | 0,1506 | 2,967 |
| 44 | 30,5 | 707 | 117 | 0,1673 | 2,929 |
| 45 | 30,9 | 596 | 59 | 0,1004 | 2,897 |
| 46 | 31,8 | 577 | 76 | 0,1338 | 2,816 |
| 47 | 32,0 | 1080 | 107 | 0,1004 | 2,796 |
| 48 | 32,5 | 512 | 51 | 0,1004 | 2,756 |
| 49 | 32,9 | 1268 | 167 | 0,1338 | 2,726 |
| 50 | 33,4 | 1180 | 117 | 0,1004 | 2,685 |

L'invention s'étend également à un procédé de préparation de la forme cristalline alpha du ranélate de strontium, caractérisé en ce que l'on porte à reflux une solution du ranélate de strontium ou de l'un de ses hydrates dans l'eau, puis on refroidit jusqu'à cristallisation complète et on recueille le produit par filtration.
- Dans le procédé de préparation selon l'invention, on peut utiliser le ranélate de strontium, ou l'un de ses hydrates, obtenu par n'importe quel procédé, par exemple l'octahydrate du ranélate de strontium obtenu par le procédé de préparation décrit dans le brevet EP 0415 850.
- L'obtention de cette forme cristalline a pour avantage de permettre une filtration particulièrement rapide et efficace, ainsi que la préparation de formulations pharmaceutiques ayant une composition constante et reproductible, ce qui est particulièrement avantageux lorsque ces formulations sont destinées à l'administration orale.
- La forme ainsi obtenue est suffisamment stable pour autoriser son stockage prolongé sans conditions particulières de température, de lumière, d'humidité ou de taux d'oxygène.

L'invention s'étend aussi aux compositions pharmaceutiques renfermant comme principe actif la forme cristalline alpha du ranélate de strontium avec un ou plusieurs excipients inertes, non toxiques et appropriés. Parmi les compositions pharmaceutiques selon l'invention, on pourra citer plus particulièrement celles qui conviennent pour l'administration orale, parentérale (intraveineuse ou sous-cutanée), nasale, les comprimés simples ou dragéifiés, les granulés, les comprimés sublinguaux, les gélules, les tablettes, les suppositoires, les crèmes, les pommades, les gels dermiques, les préparations injectables, les suspensions buvables et les pâtes à mâcher.

La posologie utile est adaptable selon la nature et la sévérité de l'affection, la voie d'administration ainsi que l'âge et le poids du patient. Cette posologie varie de 0,2 g à 10 g par jour en une ou plusieurs prises.

Les exemples suivants illustrent l'invention.

Le spectre de diffraction X sur poudre a été mesuré avec les conditions expérimentales suivantes :
- Diffractomètre PANalytical X'Pert Pro, détecteur X'Celerator,
- Tension 45 KV, intensité 40mA,
- Montage θ-θ,
- Filtre Kβ (Ni),
- fente de soller sur le faisceau incident et sur le faisceau diffracté : 0,04 rad,
- fentes de divergence : automatique, longueur irradiée : 10 mm,
- Masque : 10 mm,
- Fente anti-diffusion : 1/2°,
- Mode de mesure : continu de 3° à 34°, avec une incrémentation de 0,017°,
- Temps de mesure par pas : 31,1 s,
- Temps total : 8 min 07 s,
- Vitesse de mesure : 0,068 °/s,
- Spinner : révolution de 1 tour/s,
- Température de mesure : ambiante.

### EXEMPLE 1 : Forme cristalline alpha du ranélate de strontium

200 g de l'octahydrate du ranélate de strontium obtenu selon le procédé décrit dans le brevet EP 0415 850 sont mélangés à 2 1 d'eau et portés au reflux.
Le milieu est ensuite refroidi jusqu'à 20°C.
Le solide obtenu est collecté par filtration.

La teneur en eau du produit obtenu, déterminée par perte à la dessiccation, est de 24%, ce qui correspond à un nombre de molécules d'eau égal à 9 par molécule de ranélate de strontium.

### Diagramme de diffraction X sur poudre:

Le profil de diffraction des rayons X de la poudre (angles de diffraction) de la forme alpha du ranélate de strontium est donné par les raies significatives rassemblées dans le tableau suivant :

| **Raie n°** | **Angle 2 thêta (degrés)** | **Hauteur (coups)** | **Surface (coups x degrés)** | **FWHM (degrés)** | **Distance inter-réticulaire (Å)** |
|---|---|---|---|---|---|
| 1 | 7,6 | 4527 | 448 | 0,1004 | 11,649 |
| 2 | 8,0 | 1438 | 142 | 0,1004 | 11,069 |
| 3 | 8,3 | 3522 | 349 | 0,1004 | 10,642 |
| 4 | 8,6 | 11347 | 1123 | 0,1004 | 10,272 |
| 5 | 8,9 | 7332 | 726 | 0,1004 | 9,889 |
| 6 | 11,0 | 1047 | 104 | 0,1004 | 8,072 |
| 7 | 11,3 | 1655 | 164 | 0,1004 | 7,840 |
| 8 | 12,0 | 2186 | 216 | 0,1004 | 7,355 |
| 9 | 13,2 | 2887 | 381 | 0,1338 | 6,703 |
| 10 | 13,5 | 1705 | 169 | 0,1004 | 6,557 |
| 11 | 14,1 | 154 | 30 | 0,2007 | 6,275 |
| 12 | 14,7 | 803 | 79 | 0,1004 | 6,035 |
| 13 | 14,9 | 1346 | 178 | 0,1338 | 5,942 |
| 14 | 15,8 | 1556 | 154 | 0,1004 | 5,613 |
| 15 | 16,0 | 3339 | 441 | 0,1338 | 5,527 |
| 16 | 16,7 | 1845 | 183 | 0,1004 | 5,308 |
| 17 | 17,3 | 2835 | 281 | 0,1004 | 5,127 |
| 18 | 17,6 | 1252 | 124 | 0,1004 | 5,049 |
| 19 | 18,0 | 2183 | 216 | 0,1004 | 4,939 |
| 20 | 19,2 | 2303 | 228 | 0,1004 | 4,622 |
| 21 | 19,8 | 1298 | 128 | 0,1004 | 4,475 |
| 22 | 20,3 | 788 | 78 | 0,1004 | 4,373 |
| 23 | 20,6 | 1039 | 103 | 0,1004 | 4,317 |
| 24 | 21,1 | 882 | 116 | 0,1338 | 4,211 |
| 25 | 21,7 | 390 | 38 | 0,1004 | 4,103 |
| 26 | 22,3 | 1919 | 253 | 0,1338 | 3,990 |
| 27 | 22,7 | 1805 | 179 | 0,1004 | 3,923 |
| 28 | 23,0 | 4043 | 467 | 0,1171 | 3,861 |
| 29 | 23,5 | 650 | 86 | 0,1338 | 3,792 |
| 30 | 24,0 | 8677 | 1002 | 0,1171 | 3,711 |
| 31 | 24,7 | 229 | 30 | 0,1338 | 3,600 |
| 32 | 25,1 | 1246 | 164 | 0,1338 | 3,543 |
| 33 | 25,6 | 1659 | 219 | 0,1338 | 3,473 |
| 34 | 25,9 | 1773 | 175 | 0,1004 | 3,442 |
| 35 | 26,3 | 695 | 69 | 0,1004 | 3,385 |
| 36 | 26,6 | 401 | 46 | 0,1171 | 3,355 |
| 37 | 27,0 | 2800 | 370 | 0,1338 | 3,300 |
| 38 | 27,6 | 1415 | 140 | 0,1004 | 3,230 |
| 39 | 28,0 | 3250 | 429 | 0,1338 | 3,186 |
| 40 | 28,4 | 1513 | 250 | 0,1673 | 3,144 |
| 41 | 29,1 | 1456 | 144 | 0,1004 | 3,068 |
| 42 | 29,6 | 1943 | 192 | 0,1004 | 3,022 |
| 43 | 30,1 | 3637 | 540 | 0,1506 | 2,967 |
| 44 | 30,5 | 707 | 117 | 0,1673 | 2,929 |
| 45 | 30,9 | 596 | 59 | 0,1004 | 2,897 |
| 46 | 31,8 | 577 | 76 | 0,1338 | 2,816 |
| 47 | 32,0 | 1080 | 107 | 0,1004 | 2,796 |
| 48 | 32,5 | 512 | 51 | 0,1004 | 2,756 |
| 49 | 32,9 | 1268 | 167 | 0,1338 | 2,726 |
| 50 | 33,4 | 1180 | 117 | 0,1004 | 2,685 |

### EXEMPLE 2 : Composition pharmaceutique

Formule de préparation pour 1000 comprimés dosés à 0,5 g :

| | |
|---|---|
| Composé de l'exemple 1 | 658 g |
| Carboxyméthylamidon sodique | 25,5 g |
| Cellulose microcristalline | 119,4 g |
| Povidone | 38 g |
| Silice colloïdale anhydre | 1,5 g |
| Stéarate de magnésium | 7,6 g |

## Revendications

1. Forme cristalline alpha du ranélate de strontium de formule (I) : 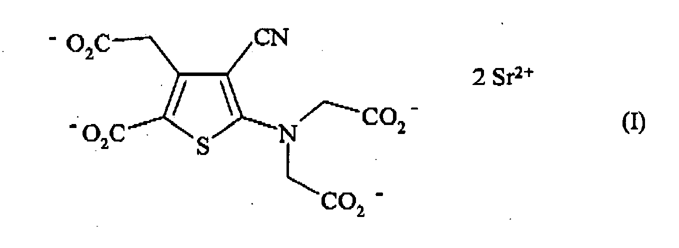 **caractérisée par** une teneur en eau de 24%, et par le diagramme de diffraction X sur poudre suivant, mesuré sur un diffractomètre PANalytical X'Pert Pro avec un détecteur X'Celerator, et exprimé en termes de position de raie (angle de Bragg 2 thêta, exprimé en degrés), de hauteur de raie (exprimée en coups), de surface de raie (exprimée en coups x degrés), de largeur des raies à mi-hauteur ("FWHM", exprimée en degrés) et de distance inter-réticulaire d (exprimée en Å) :
| Raie n° | Angle 2 thêta (degrés) | Hauteur (coups) | Surface (coups x degrés) | FHWM (degrés) | Distance inter-réticulaire (Å) |
|---|---|---|---|---|---|
| 1 | 7,6 | 4527 | 448 | 0,1004 | 11.649 |
| 2 | 8,0 | 1438 | 142 | 0,1004 | 11.069 |
| 3 | 8,3 | 3522 | 349 | 0,1004 | 10,642 |
| 4 | 8,6 | 11347 | 1123 | 0,1004 | 10,272 |
| 5 | 8.9 | 7332 | 726 | 0,1004 | 9,889 |
| 6 | 11,0 | 1047 | 104 | 0,1004 | 8,072 |
| 7 | 11.3 | 1655 | 164 | 0,1004 | 7,840 |
| 8 | 12,0 | 2186 | 216 | 0,1004 | 7,355 |
| 9 | 13.2 | 2887 | 381 | 0,1338 | 6,703 |
| 10 | 13,5 | 1705 | 169 | 0,1004 | 6,557 |
| 11 | 14.1 | 154 | 30 | 0,2007 | 6,275 |
| 12 | 14,7 | 803 | 79 | 0,1004 | 6,035 |
| 13 | 14,9 | 1346 | 178 | 0,1338 | 5,942 |
| 14 | 15,8 | 1556 | 154 | 0,1004 | 5,613 |
| 15 | 16.0 | 3339 | 441 | 0,1338 | 5,527 |
| 16 | 18,7 | 1845 | 183 | 0,1004 | 5,308 |
| 17 | 17,3 | 2835 | 281 | 0,1004 | 5,127 |
| 18 | 17,6 | 1252 | 124 | 0,1004 | 5,049 |
| 19 | 18,0 | 2183 | 216 | 0,1004 | 4,939 |
| 20 | 19,2 | 2303 | 228 | 0,1004 | 4,622 |
| 21 | 19,8 | 1298 | 128 | 0,1004 | 4,475 |
| 22 | 20.3 | 788 | 78 | 0,1004 | 4.373 |
| 23 | 20.6 | 1039 | 103 | 0,1004 | 4,317 |
| 24 | 21,1 | 882 | 116 | 0,1338 | 4,211 |
| 25 | 217 | 390 | 38 | 0,1004 | 4,103 |
| 26 | 22,3 | 1919 | 253 | 0,1338 | 3,990 |
| 27 | 22,7 | 1805 | 179 | 0,1004 | 3,923 |
| 28 | 23,0 | 4043 | 467 | 0,1171 | 3,861 |
| 29 | 23,5 | 650 | 86 | 0,1338 | 3,792 |
| 30 | 24,0 | 8677 | 1002 | 0,1171 | 3,711 |
| 31 | 24,7 | 229 | 30 | 0,1338 | 3,600 |
| 32 | 25,1 | 1246 | 164 | 0,1338 | 3,543 |
| 33 | 25,6 | 1659 | 219 | 0,1338 | 3,473 |
| 34 | 25,9 | 1773 | 175 | 0,1004 | 3,442 |
| 35 | 26,3 | 695 | 69 | 0,1004 | 3,385 |
| 36 | 26,6 | 401 | 46 | 0,1171 | 3,355 |
| 37 | 27,0 | 2800 | 370 | 0,1338 | 3,300 |
| 38 | 27,6 | 1415 | 140 | 0,1004 | 3,230 |
| 39 | 28,0 | 3250 | 429 | 0,1338 | 3,186 |
| 40 | 28,4 | 1513 | 250 | 0,1673 | 3,144 |
| 41 | 29,1 | 1456 | 144 | 0,1004 | 3,068 |
| 42 | 29,6 | 1943 | 192 | 0,1004 | 3,022 |
| 43 | 30,1 | 3637 | 540 | 0,1506 | 2,967 |
| 44 | 30,5 | 707 | 117 | 0,1673 | 2,929 |
| 45 | 30,9 | 596 | 59 | 0,1004 | 2,897 |
| 46 | 31,8 | 577 | 76 | 0,1338 | 2,816 |
| 47 | 32.0 | 1080 | 107 | 0,1004 | 2,796 |
| 48 | 32,5 | 512 | 51 | 0,1004 | 2,756 |
| 49 | 32,9 | 1268 | 167 | 0,1338 | 2,726 |
| 50 | 33,4 | 1180 | 117 | 0,1004 | 2,665 |

2. Procédé de préparation de la forme cristalline alpha du ranélate de strontium selon la revendication 1, **caractérisé en ce que** l'on porte à reflux une solution du ranélate de strontium ou de l'un de ses hydrates dans l'eau, puis on refroidit jusqu'à cristallisation complète et on recueille le produit par filtration.

3. Composition pharmaceutique contenant comme principe actif la forme cristalline alpha du ranélate de strontium selon la revendication 1, en combinaison avec un ou plusieurs véhicules inertes, non toxiques et pharmaceutiquement acceptables,

4. Utilisation de la forme cristalline alpha du ranélate de strontium selon la revendication 1, pour la fabrication de médicaments utiles dans le traitement ou la prévention de l'ostéoporose.

5. Utilisation de la forme cristalline alpha du ranélate de strontium selon la revendication 1, pour la fabrication de médicaments utiles dans le traitement ou la prévention de arthrose.

## Claims

1. Alpha crystalline form of strontium ranelate of formula (I) : 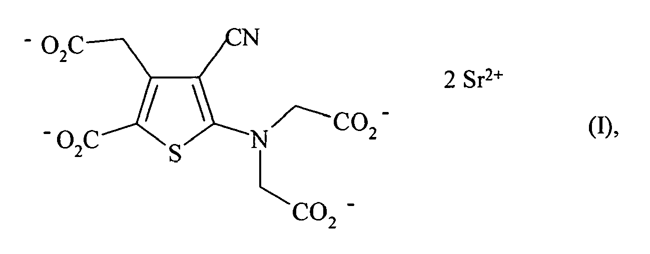 **characterised by** a water content of 24 % and by the following powder X-ray diffraction diagram measured using a PANalytical X'Pert Pro diffractometer together with an X'Celerator detector and expressed in terms of line position (Bragg's angle 2 theta, expressed in degrees), line height (expressed in counts), line area (expressed in counts x degrees), line width at half-height ("FWHM", expressed in degrees) and interplanar distance d (expressed in Å):
| **Line no.** | **Angle 2 theta (degrees)** | **Height (counts)** | **Area (counts x degrees)** | **FWHM (degrees)** | **Interplanar distance (Å)** |
|---|---|---|---|---|---|
| 1 | 7.6 | 4527 | 448 | 0.1004 | 11.649 |
| 2 | 8.0 | 1438 | 142 | 0.1004 | 11.069 |
| 3 | 8.3 | 3522 | 349 | 0.1004 | 10.642 |
| 4 | 8.6 | 11347 | 1123 | 0.1004 | 10.272 |
| 5 | 8.9 | 7332 | 726 | 0.1004 | 9.889 |
| 6 | 11.0 | 1047 | 104 | 0.1004 | 8.072 |
| 7 | 11.3 | 1655 | 164 | 0.1004 | 7.840 |
| 8 | 12.0 | 2186 | 216 | 0.1004 | 7.355 |
| 9 | 13.2 | 2887 | 381 | 0.1338 | 6.703 |
| 10 | 13.5 | 1705 | 169 | 0.1004 | 6.557 |
| 11 | 14.1 | 154 | 30 | 0.2007 | 6.275 |
| 12 | 14.7 | 803 | 79 | 0.1004 | 6.035 |
| 13 | 14.9 | 1346 | 178 | 0.1338 | 5.942 |
| 14 | 15.8 | 1556 | 154 | 0.1004 | 5.613 |
| 15 | 16.0 | 3339 | 441 | 0.1338 | 5.527 |
| 16 | 16.7 | 1845 | 183 | 0.1004 | 5.308 |
| 17 | 17.3 | 2835 | 281 | 0.1004 | 5.127 |
| 18 | 17.6 | 1252 | 124 | 0.1004 | 5.049 |
| 19 | 18.0 | 2183 | 216 | 0.1004 | 4.939 |
| 20 | 19.2 | 2303 | 228 | 0.1004 | 4.622 |
| 21 | 19.8 | 1298 | 128 | 0.1004 | 4.475 |
| 22 | 20.3 | 788 | 78 | 0.1004 | 4.373 |
| 23 | 20.6 | 1039 | 103 | 0.1004 | 4.317 |
| 24 | 21.1 | 882 | 116 | 0.1338 | 4.211 |
| 25 | 21.7 | 390 | 38 | 0.1004 | 4.103 |
| 26 | 22.3 | 1919 | 253 | 0.1338 | 3.990 |
| 27 | 22.7 | 1805 | 179 | 0.1004 | 3.923 |
| 28 | 23.0 | 4043 | 467 | 0.1171 | 3.861 |
| 29 | 23.5 | 650 | 86 | 0.1338 | 3.792 |
| 30 | 24.0 | 8677 | 1002 | 0.1171 | 3.711 |
| 31 | 24.7 | 229 | 30 | 0.1338 | 3.600 |
| 32 | 25.1 | 1246 | 164 | 0.1338 | 3.543 |
| 33 | 25.6 | 1659 | 219 | 0.1338 | 3.473 |
| 34 | 25.9 | 1773 | 175 | 0.1004 | 3.442 |
| 35 | 26.3 | 695 | 69 | 0.1004 | 3.385 |
| 36 | 26.6 | 401 | 46 | 0.1171 | 3.355 |
| 37 | 27.0 | 2800 | 370 | 0.1338 | 3.300 |
| 38 | 27.6 | 1415 | 140 | 0.1004 | 3.230 |
| 39 | 28.0 | 3250 | 429 | 0.1338 | 3.186 |
| 40 | 28.4 | 1513 | 250 | 0.1673 | 3.144 |
| 41 | 29.1 | 1456 | 144 | 0.1004 | 3.068 |
| 42 | 29.6 | 1943 | 192 | 0.1004 | 3.022 |
| 43 | 30.1 | 3637 | 540 | 0.1506 | 2.967 |
| 44 | 30.5 | 707 | 117 | 0.1673 | 2.929 |
| 45 | 30.9 | 596 | 59 | 0.1004 | 2.897 |
| 46 | 31.8 | 577 | 76 | 0.1338 | 2.816 |
| 47 | 32.0 | 1080 | 107 | 0.1004 | 2.796 |
| 48 | 32.5 | 512 | 51 | 0.1004 | 2.756 |
| 49 | 32.9 | 1268 | 167 | 0.1338 | 2.726 |
| 50 | 33.4 | 1180 | 117 | 0.1004 | 2.685 |

2. Process for the preparation of the alpha crystalline form of strontium ranelate according to claim 1, **characterised in that** a solution of strontium ranelate or a hydrate thereof in water is heated to reflux and then cooled until crystallisation is complete, and the product is collected by filtration.

3. Pharmaceutical composition comprising as active ingredient the alpha crystalline form of strontium ranelate according to claim 1 in combination with one or more pharmaceutically acceptable, inert and non-toxic carriers.

4. Use of the alpha crystalline form of strontium ranelate according to claim 1 in the manufacture of medicaments for use in the treatment or prevention of osteoporosis.

5. Use of the alpha crystalline form of strontium ranelate according to claim 1 in the manufacture of medicaments for use in the treatment or prevention of arthrosis.

## Patentansprüche

1. Alpha-Kristallform von Strontiumranelat der Formel (I): 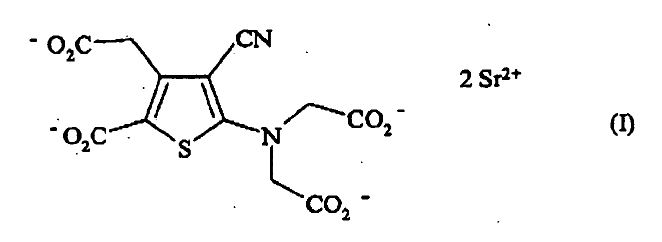 **gekennzeichnet durch** einen Wassergehalt von 24 % und das folgende Pulverröntgenbeugungsdiagramm, gemessen mit einem Diffraktometer PANalytical X'Pert Pro und einem Detektor X'Celerator und angegeben als Peakposition (Bragg-Winkel 2 Theta, angegeben in Grad), Peakhöhe (angegeben in Zählimpulsen), Peakoberfläche (angegeben in Zählimpulsen x Grad), Peakbreite bei mittlerer Höhe ("FWHM", angegeben in Grad) und Gitterabstand d (angegeben in Å):
| Peak Nr. | Winkel 2 Theta (Grad) | Höhe (Zählimpulse) | Oberfläche (Zählimpulse x Grad) | FWHM (Grad) | Gitterabstand (Å) |
|---|---|---|---|---|---|
| 1 | 7,6 | 4527 | 448 | 0,1004 | 11,649 |
| 2 | 8,0 | 1438 | 142 | 0,1004 | 11,069 |
| 3 | 8,3 | 3522 | 349 | 0,1004 | 10,642 |
| 4 | 8,6 | 11347 | 1123 | 0,1004 | 10,272 |
| 5 | 8,9 | 7332 | 726 | 0,1004 | 9,889 |
| 6 | 11,0 | 1047 | 104 | 0,1004 | 8,072 |
| 7 | 11,3 | 1655 | 164 | 0,1004 | 7,840 |
| 8 | 12,0 | 2186 | 216 | 0,1004 | 7,355 |
| 9 | 13,2 | 2887 | 381 | 0,1338 | 6,703 |
| 10 | 13,5 | 1705 | 169 | 0,1004 | 6,557 |
| 11 | 14,1 | 154 | 30 | 0,2007 | 6,275 |
| 12 | 14,7 | 803 | 79 | 0,1004 | 6,035 |
| 13 | 14,9 | 1346 | 178 | 0,1338 | 5,942 |
| 14 | 15,8 | 1556 | 154 | 0,1004 | 5,613 |
| 15 | 16,0 | 3339 | 441 | 0,1338 | 5,513 |
| 16 | 16,7 | 1845 | 183 | 0,1004 | 5,308 |
| 17 | 17,3 | 2835 | 281 | 0,1004 | 5,127 |
| 18 | 17,6 | 1252 | 124 | 0,1004 | 5,049 |
| 19 | 18,0 | 2183 | 216 | 0,1004 | 4,939 |
| 20 | 19,2 | 2303 | 228 | 0,1004 | 4,622 |
| 21 | 19,8 | 1298 | 128 | 0,1004 | 4,475 |
| 22 | 20,3 | 788 | 78 | 0,1004 | 4,373 |
| 23 | 20,6 | 1039 | 103 | 0,1004 | 4,317 |
| 24 | 21,1 | 882 | 116 | 0,1338 | 4,211 |
| 25 | 21,7 | 390 | 38 | 0,1004 | 4,103 |
| 26 | 22,3 | 1919 | 253 | 0,1338 | 3,990 |
| 27 | 22,7 | 1805 | 179 | 0,1004 | 3,923 |
| 28 | 23,0 | 4043 | 467 | 0,1171 | 3,861 |
| 29 | 23,5 | 650 | 86 | 0,1338 | 3,792 |
| 30 | 24,0 | 8677 | 1002 | 0,1171 | 3,711 |
| 31 | 24,7 | 229 | 30 | 0,1338 | 3,600 |
| 32 | 25,1 | 1246 | 164 | 0,1338 | 3,543 |
| 33 | 25,6 | 1659 | 219 | 0,1338 | 3,473 |
| 34 | 25,9 | 1773 | 175 | 0,1004 | 3,442 |
| 35 | 26,3 | 695 | 69 | 0,1004 | 3,385 |
| 36 | 26,6 | 401 | 46 | 0,1171 | 3,355 |
| 37 | 27,0 | 2800 | 370 | 0,1338 | 3,300 |
| 38 | 27,6 | 1415 | 140 | 0,1004 | 3,230 |
| 39 | 28,0 | 3250 | 429 | 0,1338 | 3,186 |
| 40 | 28,4 | 1513 | 250 | 0,1673 | 3,144 |
| 41 | 29,1 | 1456 | 144 | 0,1004 | 3,068 |
| 42 | 29,6 | 1943 | 192 | 0,1004 | 3,022 |
| 43 | 30,1 | 3637 | 540 | 0,1506 | 2,967 |
| 44 | 30,5 | 707 | 117 | 0,1673 | 2,929 |
| 45 | 30,9 | 596 | 59 | 0,1004 | 2,897 |
| 46 | 31,8 | 577 | 76 | 0,1338 | 2,816 |
| 47 | 32,0 | 1080 | 107 | 0,1004 | 2,796 |
| 48 | 32,5 | 512 | 51 | 0,1004 | 2,756 |
| 49 | 32,9 | 1268 | 167 | 0,1338 | 2,726 |
| 50 | 33,4 | 1180 | 117 | 0,1004 | 2,685 |

2. Verfahren zur Herstellung der alpha-Kristallform von Strontiumranelat nach Anspruch 1, **dadurch gekennzeichnet, dass** man eine Lösung von Strontiumranelat oder eines seiner Hydrate in Wasser zum Sieden am Rückfluss erhitzt, dann bis zur vollständigen Kristallisation abkühlt und das Produkt durch Filtration gewinnt.

3. Pharmazeutische Zubereitung enthaltend als Wirkstoff die alpha-Kristallform von Strontiumranelat nach Anspruch 1 in Kombination mit einem oder mehreren inerten, nichttoxischen und pharmazeutisch annehmbaren Hilfsstoffen.

4. Verwendung der alpha-Kristallform von Strontiumranelat nach Anspruch 1 für die Herstellung von Arzneimitteln, die nützlich sind bei der Behandlung oder Vorbeugung von Osteoporose.

5. Verwendung der alpha-Kristallform von Strontiumranelat nach Anspruch 1 für die Herstellung von Arzneimitteln, die nützlich sind bei der Behandlung oder Vorbeugung von Arthrose.
